# EUROPÄISCHE PATENTSCHRIFT

(11) **EP 2 316 417 B1**
(45) Veröffentlichungstag und Bekanntmachung des Hinweises auf die Patenterteilung: **04.10.2017**
(21) Anmeldenummer: 10164913.5
(22) Anmeldetag: 04.06.2010
(51) Int. Cl.: A61K 8/81, A61Q 19/08

(54) **Nicht-therapeutische Verwendung gegen Hautalterung**
Non-therapeutic use to prevent skin aging
Utilisation non-thérapeutique contre le vieillissement cutané

(30) Priorität: 03.09.2009 DE 102009029178
(43) Veröffentlichungstag der Anmeldung: 04.05.2011
(73) Patentinhaber: Henkel AG & Co. KGaA, 40589 Düsseldorf (DE)
(72) Erfinder: Holtkötter, Olaf, 50354, Hürth (DE); Janßen, Frank, 41470, Neuss (DE); Waldmann-Laue, Marianne, 40789, Monheim (DE); Ritschel, Ursula, 40627, Düsseldorf (DE)

(56) Entgegenhaltungen:
- EP-A1- 1 563 831
- JP-A- 2007 262 012
- JP-A- 2008 094 792
- JP-A- 2008 105 985
- JP-A- 2008 110 927
- JP-A- 2008 201 773

## Beschreibung

Die Erfindung betrifft die Verwendung bestimmter Substanzen in topischen kosmetischen oder dermatologischen Zusammensetzungen zur Hautbehandlung, die der Hautalterung und deren Anzeichen entgegenwirken.

Mittel und Behandlungsmethoden zur Behandlung reifer oder intrinsisch oder extrinsisch gealterter oder lichtgeschädigter Haut, insbesondere zur Antifaltenbehandlung, sind eine bedeutende kosmetische Herausforderung. Der Alterungsprozeß ist gekennzeichnet durch einen fortschreitenden Übergang der Hautzellen aus einem proliferativen Status in einen ruhenden, seneszenten Status. Dieser Übergang entspricht der Alterung auf zellulärer Ebene, auf den sich ein Großteil der makroskopischen Alterungseffekte zurückführen lässt. Die Zellzahl im Gewebe verringert sich und kann mangels proliferierender Zellen nicht mehr aufgefüllt werden. Dadurch können keine Reparaturen des umliegenden Gewebes durchgeführt werden, Defekte reichern sich an, wodurch die Haut atrophisch wird und erschlafft. Verstärkt wird dieser Effekt unter anderem auch durch UV-Licht, das die Haut ebenfalls belastet und deren Alterung und Erschlaffung beschleunigt.

Kosmetische Mittel gegen die Hautalterung bestehen heutzutage in der Regel aus einer Basisformulierung, die das gewünschte ansprechende Hautgefühl und eine Grundpflege für die Haut bietet, und einem Wirkstoff (oder Wirkstoffgemisch), der das Fortschreiten der Hautalterung abmildern soll. Diese Wirkstoffe oder Wirkstoffkombinationen induzieren z.B. die Neusynthese von Molekülen der extrazellulären Matrix, wie beispielsweise Kollagen, Elastin oder Hyaluronsäure, da speziell diese Moleküle in der gealterten Haut nicht mehr im ausreichenden Maße vorliegen.

Die Trennung von Basisformulierung und Wirkstoff hat den Nachteil, dass einerseits durch den zusätzlichen Einsatz einer wirksamen Menge des Wirkstoffs die Kosten der Formulierung steigen, andererseits für jede Formulierung bei Zusatz eines neuen Wirkstoffs der Wirknachweis erneut erbracht werden muss.

Der vorliegenden Erfindung lag die Aufgabe zugrunde, die oben genannten Probleme zu lösen und Zubereitungen zur Behandlung und deutlichen Reduzierung der Erscheinungsformen der Hautalterung bereitzustellen, die nicht zwingend auf der Inkorporation neuer Inhaltsstoffe beruhen.

Durch den Einsatz von Verdickern, die bereits selbst einen positiven Einfluss auf die Synthese von Molekülen der extrazellulären Matrix haben und damit als aktive Komponente klassifiziert werden können, kann die gewünschte Wirkung gegen die Hautalterung bereits mit der Basisformulierung erreicht werden.

Ein erster Gegenstand der vorliegenden Erfindung ist die nicht-therapeutische Verwendung von ganz oder teilweise neutralisierten Homopolymeren der Acrylsäure in topischen kosmetischen Mitteln zur Steigerung der zellulären Hyaluronsäure-Synthese, dadurch gekennzeichnet, dass die ganz oder teilweise neutralisierten Homopolymeren der Acrylsäure in Mengen von 0,01 bis 10 Gew.-%, jeweils bezogen auf das anwendungsfertige kosmetische Mittel, verwendet werden.

Der Einsatz von Acrylat-Copolymeren zur Verlangsamung der Hautalterung wird in der europäischen Patentanmeldung EP 1 563 831 A1 beschrieben.

Erfindungsgemäß werden ganz oder teilweise neutralisierte Homo- und/oder Copolymere der Acrylsäure in kosmetischen Mitteln zur Steigerung der zellulären Hyaluronsäure-Synthese nichttherapeutisch verwendet. Vorzugsweise erfolgt diese Verwendung in bestimmten Mengenbereichen, bezogen auf das kosmetische Mittel, so daß bevorzugte Verwendungen dadurch gekennzeichnet sind, daß die ganz oder teilweise neutralisierten Homo- und/oder Copolymeren der Acrylsäure in Mengen von 0,01 bis 5 Gew.-%, noch weiter bevorzugt von 0,05 bis 2,5 Gew.-% und insbesondere von 0,1 bis 1 Gew.-%, jeweils bezogen auf das anwendungsfertige kosmetische Mittel, verwendet werden.

Ganz bevorzugte Verwendungen erfolgen in kosmetischen Mitteln, die - bezogen auf ihr Gewicht - 0,1 Gew.-% oder 0,15 Gew.-% oder 0,2 Gew.-% oder 0,25 Gew.-% oder 0,3 Gew.-% oder 0,35 Gew.-% oder 0,4 Gew.-% oder 0,45 Gew.-% oder 0,5 Gew.-% oder 0,55 Gew.-% oder 0,6 Gew.-% oder 0,65 Gew.-% oder 0,7 Gew.-% oder 0,75 Gew.-% oder 0,8 Gew.-% oder 0,85 Gew.-% oder 0,9 Gew.-% oder 0,95 Gew.-% oder 1,0 Gew.-% ganz oder teilweise neutralisierte Homopolymere der Acrylsäure zur Steigerung der zellulären Hyaluronsäure-Synthese enthalten.

Erfindungsgemäß verwendet wird mindestens ein ganz oder teilweise neutralisiertes Homopolymer der Acrylsäure, also ein Homopolymer, das mindestens eine Struktureinheit gemäß Formel (I) enthält 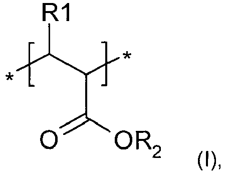 in der R1 für -H und R2 für -H oder ein einfach positiv geladenes Metallion oder eine entsprechende Gruppierung oder den n-ten Teil eines n-fach positiv geladenen Metallions oder einer entsprechenden Gruppierung steht.

Es sind erfindungsgemäße Verwendungen bevorzugt, bei denen ganz oder teilweise neutralisierte Homopolymere der Acrylsäure mit Molmassen von 200 bis 10.000 kDa, vorzugsweise von 250 bis 7500 kDa und insbesondere von 500 bis 5000 kDa verwendet werden.

Die Säurefunktion der Acrylsäure kann vollständig oder nur teilweise neutralisiert vorliegen, wobei der Neutralisationsgrad vom pH-Wert der Mittel abhängt. Vollständig nicht neutralisierte Polyacrylsäuren sind in der Regel nur unterhalb von pH-Werten zwischen 2,5 und 3 beständig, d.h. bei physiologisch verträglichen pH-Werten liegt immer ein Teil der Carboxylgruppen als Carboxylatgruppe vor. Erfindungsgemäß bevorzugte Verwendungen sind dadurch gekennzeichnet, daß der Neutralisationsgrad der ganz oder teilweise neutralisierten Homopolymere der Acrylsäure 40 bis 100%, vorzugsweise 50 bis 100%, besonders bevorzugt 60 bis 99,5% und insbesondere 70 bis 99% beträgt.

Bezogen auf den pH-Wert der Mittel, in denen die erfindungsgemäße Verwendung erfolgt, sind erfindungsgemäße Verwendungen bevorzugt, bei denen die kosmetischen Mittel pH-Werte von 4 bis 8, bevorzugt von 4,5 bis 7,5, besonders bevorzugt von 5 bis 7 und insbesondere von 5,5 bis 6,5 aufweisen.

Eine Gruppe von Polymeren, die erfindungsgemäß verwendet werden, sind Hompolymere der Acrylsäure, die ganz oder teilweise neutralisiert sind, z.B. mit Ammonium, Natrium, Kalium, Calcium oder Mischungen aus diesen Kationen. Diese Homopolymeren werden vorzugsweise alleine, d.h. nicht in Mischung mit weiteren Copolymeren der Acrylsäure eingesetzt. Andere Polymere, die keine Acrylsäure-Monomere enthalten, können selbstverständlich zugegen sein.

Erfindungsgemäß besonders bevorzugte Verwendungen sind demnach dadurch gekennzeichnet, daß ausschließlich Homopolymere ganz oder teilweise neutralisierter Polyacrylsäuren, vorzugsweise aus der Gruppe der Homopolymeren mit den INCI-Bezeichnungen Ammonium Polyacrylate und/oder Calcium Potassium Carbomer und/oder Carbomer und/oder Polyacrylic Acid und/oder Potassium Carbomer und/oder Potassium Polyacrylate und/oder Sodium Carbomer und/oder Sodium Polyacrylate verwendet werden.

Es sind erfindungsgemäße Verwendungen besonders bevorzugt, bei denen ein oder mehrere Copolymer (e) aus der Gruppe der Polymere mit den INCI-Bezeichnungen
- Ammonium Polyacrylate und/oder
- Calcium Potassium Carbomer und/oder
- Potassium Carbomer und/oder
- Potassium Polyacrylate und/oder
verwendet werden.

Die nachfolgenden Beispiele verdeutlichen den Gegenstand der Erfindung näher.

### Beispiel 1:

Untersuchung des Einflusses von Tego Carbomer auf die Hyaluronansynthese von Fibroblasten.

Humane Vorhautfibroblasten wurden mit einer Dichte von 60.000 Zellen/Vertiefung in eine 6-well-Zellkulturplatte eingesät und mit DMEM, supplementiert mit 2 % FCS, bei 37 °C und 5 % CO₂ in einer wassergesättigten Atmosphäre kultiviert.

Von einer 0,2 %-igen Lösung von Tego^{®} Carbomer 140 wurden Verdünnungen mit dem Zellkulturmedium angesetzt, sodass der Anteil der Lösung am Medium jeweils 0,1 % und 0,5 % betrug. Mit diesen Verdünnungen wurden die Fibroblasten für 24 h und 48 h kultiviert. Zur Kontrolle der Induzierbarkeit wurden Fibroblasten über den gleichen Zeitraum mit 5 ng/ml TGFβ inkubiert, als Negativkontrolle diente das normale Kulturmedium.

Die Konzentration der in das Medium abgegebenen Menge Hyaluronsäure wurde aus dem Überstand mit Hilfe des Hyaluronic Acid Quantitative Test Kit (Corgenix, vertrieben durch Genzyme Virotech GmbH) nach Herstellerangaben bestimmt. Die Extinktion wurde mit Hilfe des Spectra Max Plus Multiwellplatten Photometers bestimmt und auf Basis einer vorher vermessenen Hyaluronsäure-Standardreihe in die entsprechende Konzentration der Hyaluronsäure umgerechnet.

| | | **Extinktion** | | **HA**-**Konzentration** | |
|---|---|---|---|---|---|
| | | **Mittelwert** | **Std.abw.** | **ng/ml** | **Std**.**abw**. |
| 24 h | | | | | |
| Neg. Kontrolle | | 0,180 | 0,010 | 184,708 | 1,9 |
| Pos. Kontrolle | | 0,293 | 0,027 | 328,039 | 6,0 |
| Tego® Carbomer 140 | 0.1% | 0,199 | 0,011 | 208,282 | 29,0 |
| Tego® Carbomer 140 | 0.5% | 0,196 | 0,079 | 204,549 | 16,1 |

| 48 h | | | | | |
|---|---|---|---|---|---|
| Neg. Kontrolle | | 0,295 | 0,020 | 330,487 | 4,5 |
| Pos. Kontrolle | | 0,946 | 0,04 | 1562,344 | 16,9 |
| Tego® Carbomer 140 | 0.1% | 0,509 | 0,35 | 660,332 | 106,5 |
| Tego® Carbomer 140 | 0.5% | 0,581 | 0,19 | 786,954 | 59,8 |

Aus der Tabelle geht hervor, dass nach 24 h weder die Tego Carbomer-Lösung noch die Positivkontrolle TGFβ zu einer nennenswerten Steigerung der Hyaluronsäure-Synthese in Fibroblasten führen. Nach 48 h lässt sich allerdings eine im Vergleich zur Negativkontrolle eine deutliche Steigerung der Hyaluronsäure-Synthese sowohl bei TGFβ als auch bei der Tego Carbomer-Lösung erkennen.

## Patentansprüche

1. Nicht-therapeutische Verwendung von ganz oder teilweise neutralisierten Homopolymeren der Acrylsäure in topischen kosmetischen Mitteln zur Steigerung der zellulären Hyaluronsäure-Synthese, **dadurch gekennzeichnet, dass** die ganz oder teilweise neutralisierten Homopolymeren der Acrylsäure in Mengen von 0,01 bis 10 Gew.-%, jeweils bezogen auf das anwendungsfertige kosmetische Mittel, verwendet werden.

2. Verwendung nach Anspruch 1, **dadurch gekennzeichnet, dass** die ganz oder teilweise neutralisierten Homopolymeren der Acrylsäure in Mengen von 0,01 bis 5 Gew.-%, noch weiter bevorzugt von 0,05 bis 2,5 Gew.-% und insbesondere von 0,1 bis 1 Gew.-%, jeweils bezogen auf das anwendungsfertige kosmetische Mittel, verwendet werden.

3. Verwendung nach einem der Ansprüche 1 oder 2, **dadurch gekennzeichnet, dass** ganz oder teilweise neutralisierte Homopolymere der Acrylsäure mit Molmassen von 200 bis 10.000 kDa, vorzugsweise von 250 bis 7500 kDa und insbesondere von 500 bis 5000 kDa verwendet werden.

4. Verwendung nach einem der Ansprüche 1 bis 3, **dadurch gekennzeichnet, dass** der Neutralisationsgrad der ganz oder teilweise neutralisierten Homopolymere der Acrylsäure 40 bis 100%, vorzugsweise 50 bis 100%, besonders bevorzugt 60 bis 99,5% und insbesondere 70 bis 99% beträgt.

5. Verwendung nach einem der Ansprüche 1 bis 4, **dadurch gekennzeichnet, dass** die kosmetischen Mittel pH-Werte von 4 bis 8, bevorzugt von 4,5 bis 7,5, besonders bevorzugt von 5 bis 7 und insbesondere von 5,5 bis 6,5 aufweisen.

6. Verwendung nach einem der Ansprüche 1 bis 5, **dadurch gekennzeichnet, dass** ausschließlich Homopolymere ganz oder teilweise neutralisierter Polyacrylsäuren aus der Gruppe der Homopolymeren mit den INCI-Bezeichnungen Ammonium Polyacrylate und/oder Calcium Potassium Carbomer und/oder Carbomer und/oder Polyacrylic Acid und/oder Potassium Carbomer und/oder Potassium Polyacrylate und/oder Sodium Carbomer und/oder Sodium Polyacrylate verwendet werden.

7. Verwendung nach einem der Ansprüche 1 bis 5, **dadurch gekennzeichnet, dass** ein oder mehrere Polymere aus der Gruppe der Polymere mit den INCI-Bezeichnungen
- Ammonium Polyacrylate und/oder
- Calcium Potassium Carbomer und/oder
- Potassium Carbomer und/oder
- Potassium Polyacrylate und/oder
verwendet werden.

## Claims

1. The non-therapeutic use of completely or partially neutralized homopolymers of acrylic acid in topical cosmetic agents for increasing cellular hyaluronic acid synthesis, **characterized in that** the completely or partially neutralized homopolymers of acrylic acid are used in amounts of from 0.01 to 10 wt.%, in each case based on the ready-to-use cosmetic agent.

2. The use according to claim 1, **characterized in that** the completely or partially neutralized homopolymers of acrylic acid are used in amounts of from 0.01 to 5 wt.%, even more preferably from 0.05 to 2.5 wt.%, and in particular from 0.1 to 1 wt.%, in each case based on the ready-to-use cosmetic agent.

3. The use according to one of claims 1 or 2, **characterized in that** completely or partially neutralized homopolymers of acrylic acid having molar masses of from 200 to 10,000 kDa, preferably from 250 to 7,500 kDa, and in particular from 500 to 5,000 kDa are used.

4. The use according to one of claims 1 to 3, **characterized in that** the degree of neutralization of the completely or partially neutralized homopolymers of acrylic acid is from 40 to 100%, preferably from 50 to 100%, particularly preferably from 60 to 99.5%, and in particular from 70 to 99%.

5. The use according to one of claims 1 to 4, **characterized in that** the cosmetic agents have pH values of from 4 to 8, preferably from 4.5 to 7.5, particularly preferably from 5 to 7, and in particular from 5.5 to 6.5.

6. The use according to one of claims 1 to 5, **characterized in that** only homopolymers of completely or partially neutralized polyacrylic acids from the group of homopolymers having the INCI names Ammonium Polyacrylate and/or Calcium Potassium Carbomer and/or Carbomer and/or Polyacrylic Acid and/or Potassium Carbomer and/or Potassium Polyacrylate and/or Sodium Carbomer and/or Sodium Polyacrylate are used.

7. The use according to one of claims 1 to 5, **characterized in that** one or more polymers from the group of polymers having the INCI names
- Ammonium Polyacrylate and/or
- Calcium Potassium Carbomer and/or
- Potassium Carbomer and/or
- Potassium Acrylate
are used.

## Revendications

1. Utilisation non thérapeutique d'homopolymères, totalement ou partiellement neutralisés, de l'acide acrylique dans des compositions cosmétiques topiques pour augmenter la synthèse de l'acide hyaluronique cellulaire, **caractérisée en ce que** les homopolymères, totalement ou partiellement neutralisés, de l'acide acrylique sont utilisés dans des quantités allant de 0,01 à 10% en poids sur la base de l'agent cosmétique prêt à l'emploi.

2. Utilisation selon la revendication 1, **caractérisée en ce que** les homopolymères, totalement ou partiellement neutralisés, de l'acide acrylique sont utilisés dans des quantités allant de 0,01 à 5% en poids, encore plus préférablement de 0,05 à 2,5% en poids et en particulier de 0,1 à 1 %en poids, à chaque fois sur la base de l'agent cosmétique prêt à l'emploi.

3. Utilisation selon l'une des revendications 1 ou 2, **caractérisée en ce que** l'on utilise des homopolymères, partiellement ou totalement neutralisés, de l'acide acrylique ayant des masses molaires allant de 200 à 10000 kDa, de préférence de 250 à 7500 kDa et en particulier de 500 à 5000 kDa.

4. Utilisation selon l'une des revendications 1 à 3, **caractérisée en ce que** le degré de neutralisation des homopolymères, totalement ou partiellement neutralisés, de l'acide acrylique va de 40 à 100%, de préférence 50 à 100%, de façon particulièrement préférée de 60 à 99,5% et en particulier de 70 à 99%.

5. Utilisation selon l'une des revendications 1 à 4, **caractérisée en ce que** le pH de l'agent cosmétique va de 4 à 8, de préférence de 4,5 à 7,5, de façon particulièrement préférée de 5 à 7 et en particulier de 5,5 à 6,5.

6. Utilisation selon l'une des revendications 1 à 5, **caractérisée en ce que** l'on utilise exclusivement les homopolymères, totalement ou partiellement neutralisés, de l'acide polyacrylique du groupe des homopolymères portant les noms INCI Ammonium Polyacrylate et/ou Calcium Potassium Carbomer et/ou Carbomer et/ou Polyacrylic Acid et/ou Potassium Carbomer et/ou Potassium Polyacrylate et/ou Sodium Carbomer et/ou Sodium Polyacrylate

7. Utilisation selon l'une des revendications 1 à 5, **caractérisée en ce que** l'on utilise un ou plusieurs polymères du groupe de polymères portant les noms INCI
- Ammonium Polyacrylate et/ou
- Calcium Potassium Carbomer et/ou
- Potassium Carbomer et/ou
- Potassium Polyacrylate.
